(19) **Europäisches Patentamt**

**European Patent Office**

**Office européen des brevets**

(11) **EP 1 270 541 A1**

(12) **EUROPEAN PATENT APPLICATION**
published in accordance with Art. 158(3) EPC

(43) Date of publication:
**02.01.2003 Bulletin 2003/01**

(51) Int Cl.⁷: **C07C 68/08**, C07C 69/96

(21) Application number: **01919774.8**

(22) Date of filing: **04.04.2001**

(86) International application number:
**PCT/JP01/02925**

(87) International publication number:
**WO 01/077060 (18.10.2001 Gazette 2001/42)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **07.04.2000 JP 2000106257**
**07.04.2000 JP 2000106258**

(71) Applicant: **TEIJIN LIMITED**
**Osaka-shi Osaka 541-0054 (JP)**

(72) Inventors:
• **OHASHI, Kenji, c/o Teijin Ltd**
**Iwakuni-shi, Yamaguchi 740-0014 (JP)**
• **SUZUKI, Hirotaka, c/o Teijin Ltd**
**Iwakuni-shi, Yamaguchi 740-0014 (JP)**
• **MURAOKA, Takeshi, c/o Teijin Ltd**
**Matsuyama-shi, Ehime 791-8041 (JP)**
• **YOSHISATO, Eishin, c/o Teijin Ltd**
**Iwakuni-shi, Yamaguchi 740-0014 (JP)**
• **NAGASHIMA, Ryouichi, c/o Teijin Ltd**
**Iwakuni-shi, Yamaguchi 740-0014 (JP)**

(74) Representative: **Hallybone, Huw George et al**
**Carpmaels and Ransford,**
**43 Bloomsbury Square**
**London WC1A 2RA (GB)**

(54) **METHOD OF SEPARATING AND RECOVERING AROMATIC CARBONATE AND PRODUCTION PROCESS**

(57) A method of isolating and recovering an aromatic carbonate, in which a solvent having a solubility parameter of 7 to 10 is added to a solution of the aromatic carbonate in an aromatic hydroxy compound to precipitate the aromatic carbonate in the form of a crystal and the aromatic carbonate is isolated and recovered.

According to the present invention, there is provided an advantageous method in which a high-purity aromatic carbonate in the form of a solid can be isolated from a reaction mixture solution obtained by reacting an aromatic hydroxy compound with carbon monoxide and molecular oxygen in the presence of a catalyst. Further, a method of recovering a catalyst and a method of recycling the catalyst are provided.

EP 1 270 541 A1

Printed by Jouve, 75001 PARIS (FR)

**Description**

Detailed Description of the Invention

Technical Field of the Invention

The present invention relates to a method of isolating and recovering an aromatic carbonate. More specifically, it relates to a method in which a specific solvent is added to a solution containing an aromatic carbonate and an aromatic hydroxy compound, the mixture is subjected to crystallization to selectively crystallize the aromatic carbonate and a high-purity aromatic carbonate is isolated and recovered from the mixture.

Further, the present invention relates to a method of reacting an aromatic hydroxy compound with carbon monoxide and molecular oxygen in the presence of a catalyst to produce an aromatic carbonate, in which each of the aromatic carbonate and the catalyst is advantageously isolated from a reaction mixture solution obtained from the reaction.

Prior Art

An aromatic polycarbonate is widely used as an engineering plastic excellent in heat resistance and transparency. As a method for producing the above aromatic polycarbonate, there are generally known an interfacial polymerization method in which an aromatic dihydroxy compound and phosgene are reacted and a melt ester exchange method in which an aromatic dihydroxy compound and an aromatic carbonate are polymerized in a molten state. However, the former has a problem in the use of a large amount of a halogen-containing solvent that involves an environmental problem, such as methylene chloride or the like. In this regard, the latter has overcome the above problem. However, the problem of a decrease in polymerization activity or coloring of an aromatic polycarbonate is caused depending upon the purity of an aromatic carbonate used. Studies are therefore being made in various ways with regard to the method of isolating and recovering a high-purity aromatic carbonate from a reaction mixture solution.

Further, as a method for producing an aromatic carbonate, there are known a number of methods including a method in which an aromatic hydroxy compound is reacted with carbon monoxide and molecular oxygen in the presence of a catalyst. Generally, a carbonylation requires a complicated catalyst system, the catalyst is expensive, and further, a catalyst remaining in an aromatic carbonate causes the purity of the aromatic carbonate to decrease, so that studies are being made in various ways with regard to the isolation and recovery of an aromatic carbonate and the recycling of the catalyst system.

JP-A-6-172270 teaches a method in which a crystal adduct of diphenyl carbonate with phenol in a molar ratio of 1:1 is formed from a catalyst-containing reaction solution by suspension crystallization and it is isolated from the reaction solution. In this method, for obtaining the crystal adduct at high yields and isolating it, the concentration of the diphenyl carbonate contained in the reaction solution is limited to a narrow range of from 50 to 70 % by weight. Further, the above crystal adduct obtained by filtration contains a catalyst-containing residue, and this remaining catalyst component may cause an adverse effect such as the decomposition of the diphenyl carbonate in the subsequent purification (distillation, etc.) of the crystal adduct. Further, it is proposed to wash the crystal adduct with a mixture solution of 9 % water and 91 % phenol before the crystal adduct is heated under reduced pressure for separating a phenol. The above washing not only causes dissolution of most part of the crystal adduct, but also increases the water content in the crystal adduct. During distillation that is carried out for the purpose of isolation of the diphenyl carbonate, hydrolysis takes place and the yield of the diphenyl carbonate may be possibly decreased.

With regard to a reaction solution whose diphenyl carbonate content is smaller than 50 % by weight, it is required to carry out the operation of distillation, etc., for concentrating the reaction solution when the treatment is carried out by the above-described method. When the above operation is carried out, however, the deactivation of the catalyst under a thermal load takes place in addition to the above-described defects.

As a method utilizing suspension crystallization, there is a method disclosed in JP-A-10-45680. In this method, a reaction mixture solution containing an aromatic carbonate is cooled, and when crystallization is started, a seed material is provided to perform suspension crystallization. The resultant crystal product contains an aromatic carbonate and an aromatic hydroxy compound. The crystal product is isolated from a catalyst-containing mother liquor and then washed with a mixture of an aromatic carbonate with an aromatic hydroxy compound or with an aromatic hydroxy compound alone, and the wash solution and the catalyst-containing mother liquor are recycled to, and used in, a reaction to come thereafter. However, the crystal product obtained by the above method contains a considerable amount of an aromatic hydroxy compound. For obtaining a high-purity aromatic carbonate, therefore, the subsequent purification (distillation, etc.) for isolating the aromatic carbonate is inevitable.

In the above conventional method, the efficiency of isolation of an aromatic carbonate from a reaction mixture solution is low, so that the product quality is inevitably low and that the cost for a recovery apparatus is unavoidably large. Further, a vast amount of energy for the isolation is required.

U.S. Patent 5,981,788 proposes the following with regard to the isolation and recovery of a catalyst component from a reaction mixture obtained by the carbonylation of a phenol. That is, it is disclosed that 48 % of a palladium component, at least 90 % of a cobalt component and at least 60 % of a quaternary ammonium salt can be isolated by (1) extracting a metal component with a hydrochloric acid and sodium chloride aqueous solution at least once, (2) removing phenol by evaporation, and (3) extracting a quaternary ammonium salt with water at least once, from a reaction mixture containing phenol, diphenyl carbonate, a palladium salt, a cobalt salt and a quaternary ammonium salt. However, the above method has three disadvantages. The first disadvantage is that a cost is inevitably increased with regard to a separation apparatus and energy. The second one is that diphenyl carbonate is frequently exposed to heat, an acid and water during a process, so that the recovery is low due to hydrolysis, and the like. The third one is that a catalyst component, particularly, a quaternary ammonium salt that is easily pyrolyzable is deteriorated during heat treatment in a process. As described above, any conventional method makes it possible to isolate an aromatic carbonate and a catalyst component, but none of the conventional methods can be said to be economically advantageous.

Problems to be Solved by the Invention

It is a first object of the present invention to provide a method of isolating and recovering an aromatic carbonate from a solution containing an aromatic hydroxy compound and the aromatic carbonate by utilizing crystallization, in which inclusion of the aromatic hydroxy compound in a crystal product is avoided and the content of the aromatic carbonate in the crystal product is improved.

It is a second object of the present invention to provide a method of isolating and recovering a high-purity aromatic carbonate from a reaction mixture solution obtained by carbonylation of an aromatic hydroxy compound, according to a crystallization method.

Further, it is a third object of the present invention to provide an industrially advantageous method in which not only an aromatic carbonate is recovered from the above reaction mixture solution obtained by carbonylation, but also a used catalyst is recovered from the above reaction mixture solution obtained by carbonylation at high yields without decreasing its activity and can be recycled.

It is further another object of the present invention to provide an advantageous method of removing water formed by a reaction from the above reaction mixture solution obtained by carbonylation. According to the method of removing water from the reaction mixture solution, provided by the present invention, water can be removed under moderate conditions and by simple means, so that the hydrolysis of an aromatic carbonate can be inhibited and that a decrease in the activity of a catalyst can be remarkably prevented.

Means to Solve the Problems

First, the present inventors have made studies for methods for isolating a high-purity aromatic carbonate, as a crystal, from a solution containing an aromatic hydroxy compound and an aromatic carbonate by a crystallization method.

The crystallization method will be explained below with respect to a case where diphenyl carbonate (to be referred to as "DPC" hereinafter) is selected as a diaryl carbonate and phenol is selected as an aromatic hydroxy compound. However, the present invention shall not be limited thereto.

The present inventors started studies with comparing DPC and phenol in solubility while employing solubility parameters introduced from the concept of mole attraction force of K. L. Hoy (J. Paint Technol., 42,76) (1970). Hoy defines a mole attraction constant G determined by vapor pressure measurement with regard to several atoms and atomic groups, and it is said that a value of solubility parameter ($\delta$) can be estimated from molecular structural formula on the basis of the following equation,

$$\delta = d\Sigma G/M$$

wherein $\delta$ is a solubility parameter, d is a density, G is a mole attraction constant and M is a molecular weight. For further details of calculation of the value of the solubility parameter ($\delta$), the original literature can be referred to.

When solubility parameters of DPC and phenol are calculated on the basis of the above equation, the solubility parameter $\delta_{dpc}$ (suffixes dpc stands for DPC) of DPC comes to be 10.25, and the solubility parameter $\delta_{ph}$ (suffixes ph stands for phenol) of phenol comes to be 9.73. Concerning values of the density (d), values at $20°C$ are used. These values differ from each other by 0.52 or are close to each other, which means that these two components are easily compatible to each other. This result endorses a difficulty in isolating DPC from a mixture solution of these two components by crystallization operation according to a crystallization method.

The present inventors have therefore continued studies for isolating DPC by a crystallization method. As a result, it has been found that when a specific amount of a third-component solvent having a specific solubility parameter is added to the above solution, the third-component solvent produces an effect that the solubility parameter $\delta_{ph}$ of phenol is apparently decreased. Further surprisingly, it has been found that the amount of phenol occluded in or adsorbed on DPC, which is a conventional problem, is remarkably decreased. The present invention has been arrived at on the basis of the above findings.

That is, according to the present invention, there is provided a method of isolating and recovering an aromatic carbonate from a solution (A), of an aromatic carbonate of the following formula (I),

$$R\text{-}O\text{-}CO\text{-}O\text{-}R \tag{I}$$

wherein R is an aromatic hydrocarbon group having 6 to 15 carbon atoms, the aromatic hydrocarbon group having a substituent or having no substituent,
and an aromatic hydroxy compound of the following formula (II),

$$R\text{-}OH \tag{II}$$

wherein R is as defined in the formula (I),
which comprises adding a solvent (B) having a solubility parameter ($\delta_s$) in the range of from 7.0 to 10.0 to the solution (A) selectively precipitating the aromatic carbonate in said solution (A), and then recovering the aromatic carbonate.

According to the present invention, further, there is provided a method for producing an aromatic carbonate of the following formula (I),

$$R\text{-}O\text{-}CO\text{-}O\text{-}R \tag{I}$$

wherein R is an aromatic hydrocarbon group having 6 to 15 carbon atoms, the aromatic hydrocarbon group having a substituent or having no substituent,
by reacting an aromatic hydroxy compound of the following formula (II),

$$R\text{-}OH \tag{II}$$

wherein R is as defined in the formula (I),
with carbon monoxide and molecular oxygen in the presence of a catalyst,
　　the method further comprising adding a solvent (B) having a solubility parameter ($\delta_s$) in the range of from 7.0 to 10.0 to a reaction mixture solution (A') obtained by said reaction, selectively precipitating the aromatic carbonate in said reaction mixture solution (A') and then, recovering the aromatic carbonate from said reaction mixture solution (A').

Further, according to the present invention, there is provided a method comprising evaporating water under heat insulation in the above reaction mixture solution (A') by flashing the above solution (A'), to obtain a solution (A") and isolating and recovering the aromatic carbonate from the solution (A"), and there is also provided a method comprising adding a solvent (B) having a solubility parameter $\delta_s$ in the range of from 7.0 to 10.0 to the above solution (A"), thereby selectively precipitating an aromatic carbonate and then isolating and recovering the aromatic carbonate.

The method of the present invention will be further specifically explained hereinafter.

In the present invention, the aromatic hydroxy compound refers to a compound of the above formula (II). In the above formula (II), R is an aromatic hydrocarbon group which has a substituent or does not have any substituent and which has 6 to 15 carbon atoms, preferably 6 to 12 carbon atoms. The aromatic hydrocarbon group is preferably phenyl or naphthyl, particularly preferably phenyl. When the aromatic hydrocarbon group (R) has a substituent, examples of the substituent include an alkyl group having 1 to 4 carbon atoms, an alkoxy group having 1 to 4 carbon atoms, a halogen atom and a carboxyl group. The number of the substituent(s) is 1 to 4, preferably 1 or 2. Specific examples of the aromatic hydroxy compound include phenol, substituted phenols such as cresol, xylenol, ethylphenol, propylphenol, methoxyphenol, ethoxyphenol, chlorophenol, bromophenol and salicylic acid, isomers thereof; naphthol, substituted naphthols such as methylnaphthol and chloronaphthol, and isomers thereof. Of these, phenol is particularly preferred.

In the present invention, the aromatic carbonate refers to a compound of the above general formula (I). In the above formula (I), R is a hydrocarbon group that is the same as the aromatic hydrocarbon group defined in the explanation of the aromatic hydroxy compound of the above general formula (II). Those groups that are shown to be preferred as R therein are similarly preferred. R is most preferably a phenyl group. Specifically, the aromatic carbonate is diphenyl carbonate or dinaphthyl carbonate, particularly preferably diphenyl carbonate.

In the present invention, the aromatic carbonate contained in the aromatic hydroxy compound is precipitated, and it is isolated and recovered in the form of a solid crystal. In this case, a solvent (B) having a specific solubility parameter ($\delta_s$) is added to the above solution.

In the solution (A) of the aromatic carbonate in the aromatic hydroxy compound, the concentration (content) of the aromatic carbonae is in the range of from 5 to 95 % by weight, preferably in the range of from 7 to 70 % by weight, particularly preferably in the range of from 10 to 50 % by weight.

The solvent (B) to be added to the above solution (A) is a compound having a solubility parameter ($\delta_s$) in the range of from 7.0 to 10.0. The addition of the solvent (B) can apparently decrease the melting point of the aromatic hydroxy compound, and the crystallization temperature range of the aromatic carbonate in the present invention can be broadened. The melting point of the solvent (B) per se is preferably lower than the melting point of the aromatic hydroxy compound. The melting point of the solvent (B) is generally 41°C or lower, more preferably 15°C or lower, which is advantageous.

By adding the solvent (B) to the above solution (A), the eutectic point of the aromatic carbonate and the aromatic hydroxy compound shifts to a low-temperature side, and the concentration of the aromatic carbonate shifts toward a lower concentration side of the aromatic carbonate content, so that the aromatic carbonate precipitates from the above solution (A) and can be isolated.

The solvent (B) for use in the method of the present invention is preferably at least one selected from the group consisting of aliphatic and alicyclic hydrocarbons having 5 to 8 carbon atoms, an aromatic hydrocarbon having 6 to 8 carbon atoms, aliphatic and alicyclic ethers having 3 to 6 carbon atoms, aliphatic and alicyclic ketones having 3 to 6 carbon atoms and an aliphatic alcohol having 1 to 4 carbon atoms.

Specifically, the above solvent (B) is advantageously at least one selected from the group consisting of cyclohexane, toluene, xylene, diethyl ether, tetrahydrofuran, 1,2-dimethoxyethane, methyl-t-butyl ether, acetone, methyl ethyl ketone, methyl isobutyl ketone, methyl alcohol, ethyl alcohol and isopropyl alcohol.

According to studies by the present inventors, it has been found that a high-purity aromatic carbonate in the form a solid can be isolated and recovered when the above solvent (B) is added to the above solution (A) in such an amount ratio that the following equations (III) and (IV) are satisfied.

$$\delta_{sah} = \delta_s W_s/(W_s + W_{ah}) + \delta_{ah} W_{ah}/(W_s + W_{ah}) \tag{III}$$

$$\delta_{dac} - \delta_{sah} \geqq 0.6 \tag{IV}$$

In the equations (III) and (IV), symbols have the following meanings.

$\delta_s$: Solubility parameter of solvent (B)
$\delta_{ah}$: Solubility parameter of aromatic hydroxy compound
$\delta_{sah}$; Solubility parameter of solution (A)
$\delta_{dac}$: Solubility parameter of aromatic carbonate
$W_s$: Amount (weight) of solvent (B)
$W_{ah}$: Amount (weight) of aromatic hydroxy compound in solution (A)

When the solvent (B) is added to the solution (A), it is preferred to practice the addition with stirring, for the operation of crystallization of the aromatic carbonate and for decreasing the occlusion of impurities into a crystal. Since, however, the addition of the solvent (B) to the solution (A) improves the homogeneity in the solution, the stirring operation not particularly required, and the solvent (B) may be added in a state where the solution (A) is allowed to stand still.

According to studies by the present inventors, it has been found that the method of isolating the aromatic carbonate from the solution (A) by precipitation in the above present invention can be advantageously applied to the isolation of an aromatic carbonate from a reaction mixture solution obtained in the synthesis of an aromatic carbonate to be explained below.

That is, according to the present invention, there is provided a method for producing an aromatic carbonate of the above formula (I) by reacting an aromatic hydroxy compound of the above general formula (II) with carbon monoxide and molecular oxygen in the presence of a catalyst, the method comprising adding the solvent (B) having

a solubility parameter $\delta_s$ in the range of from 7.0 to 10.0 to a reaction mixture solution (A') obtained by the above reaction, selectively precipitating the aromatic carbonate contained in said reaction mixture solution (A') and then recovering the aromatic carbonate.

While the above reaction mixture solution (A') contains the catalyst and water formed by the reaction in addition to the aromatic carbonate and the aromatic hydroxy compound, the aromatic carbonate can be isolated and recovered in the form of a solid by adding the solvent (B) to the above reaction mixture solution (A'). Further, the solution containing the catalyst, obtained after the separation of the aromatic carbonate from the reaction mixture solution (A') can be recycled to a reaction.

The catalyst for use in the synthesis of the aromatic carbonate is desirably a catalyst system containing (a) a platinum group metal or a platinum group metal compound, (b) a redox agent and (c) a quaternary ammonium salt or a quaternary phosphonium salt.

Examples of the (a) platinum group metal in the above catalyst system include palladium, platinum, ruthenium, rhodium, osmium and iridium. Of these, palladium is particularly preferred. The state of the above platinum group metal is a metal state having activity, an inorganic acid salt such as palladium nitrate or palladium chloride, an organic acid salt such as palladium acetate, a complex such as palladium acetylacetonate, an oxide, a hydroxide or a form of a complex compound containing, for example, carbon monoxide, an olefin, an amine, a phosphine or a halogen. Further, the platinum group metal may be in a state where any one of the above platinum metal compounds is supported on an appropriate substrate such as activated carbon, silica gel, a metal oxide such as alumina, titanium oxide or zirconium oxide, a metal complex oxide such as perovskite or silicon carbide. The platinum group metal may be in a state where the above support and most part thereof are separated and removed. Above all, palladium acetylacetonate is preferred.

As the above (a) platinum group metal or the compound thereof, palladium metal or a compound thereof is the most preferred. When palladium or a palladium compound is used, properly, the amount thereof, as palladium atom, per mole of the aromatic hydroxy compound is in the range of from 1 to $1 \times 10^{-5}$ mol, preferably from $1 \times 10^{-2}$ to $1 \times 10^{-4}$ mol.

The (b) redox agent for use in the above catalyst system includes compounds of elements coming under Groups IIIA, IVA, VA, VIA, IB, IIB, VIB, VIIB, iron group (VIII) and rare earth metal group (IIIB) of the periodic table. Compounds of these metals can be used in various oxidized states, and for example, they can be used in the form of a halide, an oxide, a hydroxide, a carbonate, an organic carboxylate or a diketone salt, or in the form of a complex salt of an oxalate or salicylate. Further, they can be used in the form of a complex having, as a ligand, carbon monoxide, an olefin, an amine or a phosphine. Of these metal compounds having redox capability, a compound of manganese, cobalt, copper or a rare earth metal such as lanthanum or cerium and a compound of lead are preferred, and compounds of manganese, cobalt, copper, cerium and lead are particularly preferred. While the amount of the (b) redox agent for use is not critical, the amount thereof per mole of the (a) platinum group metal or platinum group metal compound is preferably in the range of from $1 \times 10^{-1}$ to $1 \times 10^3$ mol, particularly preferably in the range of from $1 \times 10^{-1}$ to x $10^2$ mol.

Further, the (c) quaternary ammonium salt or quaternary phosphonium salt in the above catalyst system can be selected from those of the following formula (V) or (VI).

$$R_1R_2R_3R_4NX \qquad\qquad (V)$$

$$R_1R_2R_3R_4PX \qquad\qquad (VI)$$

In these formulae, each of $R_1$ to $R_4$ is $C_1$ - $C_8$ alkyl or $C_6$ - $C_{12}$ aryl and may be the same as every other one or different from any other. X is an anion and is preferably selected from a hydroxyl group, an alkoxy group, a phenoxy group or halogen such as chloride, bromide or iodide.

Of these, tetra-n-butylammonium salt and tetraphenylphosphonium salt are particularly preferred. The amount of the quaternary ammonium salt or the quaternary phosphonium salt for use in the reaction per mole of the platinum group metal or a compound thereof, particularly, palladium or a palladium compound, is preferably in the range of from 0.1 to 1,000 mol, particularly preferably in the range of from 1 to 100 mol.

In the present invention, the aromatic hydroxy compound is reacted with carbon monoxide and molecular oxygen in the presence of the above catalyst system, whereby a diaryl carbonate as an end product can be obtained. As a catalyst that exhibits a reaction-promoting effect, (d) a heteropolyacid may be used.

The (d) hereropolyacid is a polyacid formed by dehydrative condensation of two or more oxygen acids (oxyacids) and is formed of a negatively charged oxide molecule cluster (polyanion) and a proton.

As a heteropolyacid, those having various compositions-structures are known, and any one of them can be used. Of these, a heteropolyacid having a structure called Keggin structure and having an anion portion of which the structural formula is represented by the following general formula (VII),

$$XM_{12}O_{40} \qquad (VII)$$

is preferred. In the above formula, X stands for P or Si element or other element such as As, Ge, B or the like, and M is Mo or W but is partly replaced with an atom such as V, Mn, Co, Cu, Fe, Zn or the like. Those having a proton in place of the above anion are called polyacids, and those formed by replacing the proton with a cation are also effective in the present invention. The above cation may be a metal ion of an alkali metal such as Li, Na, K, Rb, Cs or the like, and in addition thereto, it may be a metal ion of an alkaline earth metal such as Ca, Mg or the like, metal ion such as Cu, Zn, Al or the like, or a transition metal ion of Fe, Co, Ni, Mn, Cr, or the like. Or, it may be in the form of a salt of a rare earth metal ion of Ce, La, or the like. Further, it may be in the form of a salt soluble in an organic solvent, such as an ammonium salt, an organic ammonium salt, an organic phosphonium salt, or the like.

Of those heteropolyacids having a Keggin structure, particular preferred is a heteropolyacid having a Keggin structure of which the anion portion is represented by the following structural formula (VIII),

$$X(Mo)_n(W)_m(V)_pO_{40} \qquad (VIII),$$

wherein X is a P or Si element, each of n, m and p is a numerical value of 0 to 12 and has the relationship of n + m + p = 12, or salts thereof.

Specific examples thereof include phosphorus tungstomolybdic acids (such as $PMo_2W_{10}O_{40}$, $PMo_4W_8O_{40}$, $PMo_6W_6O_{40}$, $PMo_8W_4O_{40}$ and $PMo_{10}W_2O_{40}$), phosphorus vanadomolybdic acids (such as $PMo_{11}V_1O_{40}$, $PMo_{10}V_2O_{40}$. $PMo_9V_3O_{40}$, $PMo_4V_8O_{40}$ and $PMo_2V_{10}O_{40}$), phosphorus vanadotungstic acids (such as $PW_9V_3O_{40}$, $PW_{10}V_2O_{40}$ and $PW_{11}V_1O_{40}$), silimolybdotungstic acids (such as $SiMo_3W_9O_{40}$, $SiMo_6W_6O_{40}$ and $SiMO_8W_4O_{40}$), silivanadotungstic acids (such as $SiW_{11}V_1O_{40}$, $SiW_{10}V_2O_{40}$, $SiW_9V_3O_{40}$, $SiW_8V_4O_{40}$, $SiW_6V_6O_{40}$ and $SiW_8V_4O_{40}$), and silimolybdotungstic acids (such as $SiMo_{11}V_1O_{40}$, $SiMO_{10}V_2O_{40}$, $SiMo_9V_3O_{40}$, $SiMo_8V_4O_{40}$, $SiMo_6V_6O_{40}$ and $SiMo_8V_4O_{40}$). These heteropolyacids may be used alone or in the form of a mixture containing at least two of them. Further, they may be used in the form of salts of various cations or mixtures thereof as described above.

For carrying out the above reaction, any one of a closed method and a gas flow method may be employed. When the pressure of carbon monoxide and molecular oxygen is taken as a total pressure, the pressure inside the reactor is 0.1 to 1 MPa, preferably 0.5 to 0.9 MPa. The ratio of the oxygen partial pressure to the total pressure is not specially limited so long as it is outside the range of oxygen explosion. Further, when a flow method is employed, the gas flow rate of carbon monoxide per mole of the aromatic hydroxy compound is in the range of from 0.3 to 2.0 L/minute, and that of the molecular oxygen is in the range of from 0.01 to 0.1 L/minute. Preferably, the flow rate of the carbon monoxide is 0.75 to 1.2 L/minute, and that of the molecular oxygen is 0.03 to 0.075 L/minute.

While the temperature for the above reaction is not critical, it is preferably in the range of from 60°C to 120°C, more preferably, from 800C to 100°C.

In the reaction, further, an organic solvent inert to the reaction may be used. The organic solvent is preferably an aliphatic ether compound having a molecule containing one to several ether bonds. Such organic solvents having a boiling point of 30 to 170°C in particular are preferred. As a solvent, particularly preferred is tetrahydrofuran, methyl-t-butyl ether or 1,2-dimethoxyethane.

The aromatic hydroxy compound is reacted with carbon monoxide and molecular oxygen in the presence of the above catalyst, to produce an aromatic carbonate, and the thus-obtained reaction mixture solution (A') containing, as main components, the aromatic carbonate and aromatic hydroxy compound is introduced to a proper crystallizer. The concentration of the aromatic carbonate in the obtained reaction mixture solution (A') is 5 to 95 % by weight, preferably 7 to 70 % by weight.

The crystallizer for use in the present invention includes a stirrer crystallizer described in JP-A-10-45680 and a bundled-tube crystallizer described in JP-A-10-59904, although the crystallizer shall not be limited thereto.

In the present invention, a solvent (B) having a solubility parameter $\delta_s$ in the range of from 7.0 to 10.0 is added to the reaction mixture solution (A') obtained by the above reaction, and the aromatic carbonate in the reaction mixture solution (A') is selectively precipitated and recovered in the form of a solid. In this operation, desirably, the reaction mixture solution (A') after the addition of the solvent (B) is cooled from a temperature in the range of from 80 to 40°C, preferably 60 to 40°C, to a temperature in the range of from 30 to -30°C, preferably 30 to 0°C. By cooling with the solution temperature and also with the activity of the added solvent (B), the crystallization of the aromatic carbonate is promoted, and a high-purity aromatic carbonate can be obtained.

The solvent (B) to be added to the reaction mixture solution (A') is the same as the already described solvent, and it is preferably added in such an amount ratio that the already described equations (III) and (IV) are satisfied.

The aromatic carbonate that has precipitated as a crystal can be separated by centrifugal separation or filtering. The solution remaining after the separation contains the catalyst, and the catalyst can be recovered by removing the solvent. For removing the solvent, distillation is carried out under moderate conditions, which is desirable in that the decrease of the catalyst activity is prevented. The recovered catalyst is recycled to the reactor and used for the synthesis of an aromatic carbonate.

In carrying out the method of isolating and recovering the aromatic carbonate from the above reaction mixture solution (A') in the form of a solid by crystallization, the present inventors has continued to make studies for a method of effectively removing water contained in the reaction mixture solution (A'). The presence of water causes the aromatic carbonate to be hydrolyzed, and it causes the yield of the aromatic carbonate obtained by crystallization to decrease and also causes the purity thereof to decrease. Further, when attempts are made to remove water from the reaction mixture solution by the operation of usual distillation, a thermal load is caused on the catalyst, and as a result, the catalyst activity is decreased.

The present inventors have therefore found that the method of evaporating water under heat insulation by flashing a reaction mixture solution is remarkably effective for removing water contained in the reaction mixture solution (A').

According to the present invention, there is accordingly provided a method for producing an aromatic carbonate, which comprises evaporating water in the reaction mixture solution (A') obtained by the above reaction, under heat insulation by flashing said solution, to obtain a solution (A"), and isolating and recovering an aromatic carbonate from the solution (A").

The above reaction mixture solution (A') immediately after the reaction generally has a temperature of from 60 to 120°C, preferably from 80 to 120°C, and the reaction mixture solution (A') under such a high temperature is flashed to evaporate water under heat insulation, whereby water is mainly removed. The reaction mixture solution (A') is supplied to a container for evaporation under heat insulation, and flashed. The temperature and pressure conditions of the above container are set so that water is mainly evaporated from the reaction mixture solution (A') under heat insulation. Specifically, the temperature inside the container is properly in the range of from 0 to 100°C, preferably from 0 to 80°C. The pressure is properly 1 MPa or lower, preferably 0.1 MPa or lower, particularly preferably in the range of from 0.005 to 0.07 MPa. It is not necessarily required to completely remove water contained in the reaction mixture solution (A') by evaporation under heat insulation. For example, even if water in an amount of 10 % by weight or less, preferably 5 % by weight or less based on the content of water that has been contained in the reaction mixture solution (A') remains in the solution, there is no special difficulty in operations to be carried out later.

By removing water from the above reaction mixture solution (A') by evaporation under heat insulation, the solution (A") containing substantially no water is obtained. The above solution (A") contains the aromatic carbonate having a concentration of 5 to 95 % by weight, preferably 7 to 70 % by weight, more preferably 10 to 50 % by weight, which is desirable for the recovery to be described later.

For isolating and recovering the aromatic carbonate from the above solution (A"), according to the present invention already explained, the solvent (B) having a solubility parameter $\delta_s$ in the range of from 7.0 to 10.0 is added to the solution (A") to selectively precipitate the aromatic carbonate in the solution (A"), and then the aromatic carbonate can be separated and recovered.

Further, the solvent (B) having a solubility parameter $\delta_s$ in the range of from 7.0 to 10.0 is added to the above solution (A") to selectively precipitate the aromatic carbonate in the solution (A"), and then the aromatic carbonate is separated and recovered. On the other hand, a solution (C) that remains and contains the catalyst can be recycled to a reaction.

In the above precipitation of the aromatic carbonate, desirably, the solution (A") after the addition of the solvent (B) is cooled from a temperature in the range of from 80 to 40°C, preferably from 60 to 40°C, to a temperature in the range of from 30 to -30°C, preferably from 30 to 0°C. By cooling with the solution (A") temperature and also with the activity of the added solvent (B), the crystallization of the aromatic carbonate is promoted, and a higher-purity aromatic carbonate can be obtained.

The solvent (B) to be added to the solution (A") is the same as the already described solvent, and it is preferably added in such an amount ratio that the already described equations (III) and (IV) are satisfied.

The aromatic carbonate that has precipitated as a crystal can be separated by centrifugal separation or filtering. The solution remaining after the separation contains the catalyst, and the catalyst can be recovered by removing the solvent. For removing the solvent, distillation is carried out under moderate conditions, which is desirable in that the decrease of the catalyst activity is prevented. The recovered catalyst is recycled to the reactor and used for the synthesis of an aromatic carbonate.

Examples

The present invention will be explained with reference to Examples hereinafter, while the present invention shall not be limited thereto.

Example 1

A cylindrical vertical glass container (empty volume 50.0 cc) was used as a crystallizer, and the container had no stirrer. Into the container were introduced 10.0 g of a DPC powder ($\delta_{dac}$ = 10.25) and 10.0 g ($\delta_{ah}$ = 9.73) of a phenol powder. Then, the container was immersed in an oil bath and heated up to 50.0°C, to melt these powders. The resultant solution had a DPC concentration of 50 % by weight. Under standing conditions, 4.00 g of tetrahydrofuran ($\delta_s$ = 7.91, melting point: -109°C) was added to the above solution, and the solution of the melt was gradually cooled from 45.0°C to 20.0°C. In this case, according to the above equation (III), $\delta_{sah}$ = 9.21, and the above equation (IV) was satisfied. Upon completion of crystallization, a crystal and a residue were withdrawn from an outlet in the lower portion of the container, and the crystal was separated through a membrane filter. The crystal obtained had a weight of 7.70 g. DPC contained in the obtained crystal was quantitatively determined by gas chromatography, and as a result, the obtained crystal had a DPC concentration of 98.3 % by weight. The recovery of DPC was therefore 75.7 % by weight.

Example 2

A cylindrical vertical glass container (empty volume 50.0 cc) was used as a crystallizer like Example 1, and the container had an anchor agitator. Into the container were introduced 10.0 g of a DPC powder ($\delta_{dac}$ = 10.25) and 10.0 g ($\delta_{ah}$ = 9.73) of a phenol powder. Then, the container was immersed in an oil bath and heated up to 50.0°C, to melt these powders. The resultant solution had a DPC concentration of 50 % by weight. Under standing conditions, 4.00 g of acetone ($\delta_s$ = 7.59, melting point: -94.7°C) was added to the solution, and while the solution of the melt was stirred at 10 rpm, the solution was gradually cooled from 45.0°C to 20.0°C. In this case, according to the above equation (III), $\delta_{sah}$ = 9.12, and the above equation (IV) was satisfied. Upon completion of crystallization, a crystal and a residue were withdrawn from an outlet in the lower portion of the container, and the crystal was separated through a membrane filter. The crystal obtained had a weight of 6.11 g. DPC contained in the obtained crystal was quantitatively determined by gas chromatography, and as a result, the obtained crystal had a DPC concentration of 96.5 % by weight. The recovery of DPC was therefore 58.9 % by weight.

Example 3

A cylindrical vertical glass container having an empty volume of 50.0 mL was used as a crystallizer like Example 1, and the container had an anchor agitator. Into the container were introduced 6.0 g of a DPC powder ($\delta_{dac}$ = 10.25) and 14.0 g ($\delta_{ah}$ = 9.73) of a phenol powder. Then, the container was immersed in an oil bath and heated up to 50.0°C, to melt these powders. The resultant solution had a DPC concentration of 30 % by weight. While the solution was stirred at 10 rpm, acetone ($\delta_s$ = 7.59, melting point: -94.7°C) was added to the solution, and the solution was gradually cooled from 45.0°C to 20.0°C. The amount of tetrahydrofuran added was 4.00 g (According to the above equation (III), $\delta_{sah}$ = 9.26, and the range defined by the above equation (IV) was satisfied.) Upon completion of crystallization, a crystal and a residue were withdrawn from an outlet in the lower portion of the container, and the crystal was separated through a membrane filter. The crystal obtained had a weight of 4.20 g. DPC contained in the obtained crystal was quantitatively determined by gas chromatography, and as a result, the obtained crystal had a DPC concentration of 96.1 % by weight. The recovery of DPC was therefore 67.3 % by weight.

Example 4

An autoclave made of stainless steel having an empty volume of 500 mL was charged with 0.200 g of palladium acetylacetonate, 250 g of phenol, 0.500 g of lead oxide, 0.350 g of manganese acetylacetonate and 5.00 g of tetrabutylammonium bromide. The temperature inside the container was maintained at 80.0°C, and an atmosphere in the container was substituted consecutively with nitrogen and carbon monoxide. Further, the pressure inside the container was increased up to 0.780 MPa with carbon monoxide. Then, carbon monoxide and oxygen were concurrently flowed at a carbon monoxide flow rate of 2.50 L/minute and at an oxygen flow rate of 0.125 L/minute, to start a reaction. The reaction was continued for 3 hours, and as a result, DPC was generated at 17.9 % based on phenol. 20.0 Grams (DPC content: 3.97 g) of the thus-obtained reaction mixture was collected, and introduced into a cylindrical vertical glass container (empty volume 50.0 cc) having no stirrer. Further, 10.0 g of tetrahydrofuran ($\delta_s$ = 7.91, melting point: -109°C) was added, and the mixture was gradually cooled from 45.0°C to 20.0°C. In this case, $\delta_{dac}$ - $\delta_{sah}$ = 1.22. Upon com-

pletion of crystallization, a crystal and a residue were withdrawn from an outlet in the lower portion of the container, and the crystal was separated through a membrane filter. The crystal obtained had a weight of 3.22 g. DPC contained in the obtained crystal was quantitatively determined by gas chromatography, and as a result, the obtained crystal had a DPC concentration of 98.0 % by weight. The recovery of DPC was therefore 79.4 % by weight. Further, concerning the amount of tetrabutylammonium bromide contained in the residue, silver nitrate was added to precipitate silver bromide, and the precipitate was weighed to determine the amount thereof. The recovery thereof was calculated. As a result, the recovery of the tetrabutylammonium bromide was 98.8 % by weight. A solution containing the catalyst, obtained upon completion of the crystallization, was recycled, to show that DPC was stably produced.

Example 5

Example 4 was repeated except that the solvent added was replaced with 10.0 g of methyl alcohol ($\delta_s$ = 9.24, melting point: -97.5°C). Further, as a reaction mixture solution, 20.0 g of a reaction mixture solution (having a DPC content of 3.97 g and having a DPC purity of 19.9 % by weight) was collected from the reaction mixture solution obtained in Example 4 and used. A crystal obtained had a weight of 2.89 g, and DPC had a concentration of 99.0 % by weight. The recovery of DPC was therefore 72.0 % by weight. Further, the recovery of tetrabutylammonium bromide was 99.5 % by weight. Moreover, a solution containing the catalyst, obtained upon completion of the crystallization, was recycled, to show that DPC was stably produced.

Example 6

An autoclave made of stainless steel having an empty volume of 100 mL was charged with 20.0 g of phenol, 30.0 g of 1,2-dimethoxyethane ($\delta_s$ = 7.48, melting point: - 58.0°C), 0.0200 g of palladium acetylacetonate, 0.0350 g of manganese acetylacetonate, 0.0540 g of a heteropolyacid (tetra-n-butyl silitungsto-11-molybdate ammonium salt) and 0.400 g of tetrabutylammonium bromide. The temperature inside the container was maintained at, 80.0°C, and an atmosphere in the autoclave was substituted consecutively with nitrogen and carbon monoxide. Further, the pressure inside the container was increased up to 0.780 MPa with carbon monoxide. Then, carbon monoxide and oxygen were concurrently flowed at a carbon monoxide flow rate of 1.00 L/minute and at an oxygen flow rate of 0.0700 L/minute, to start a reaction. While the partial pressures of carbon monoxide and molecular oxygen were maintained at a constant rate, the reaction was continued for 9 hours, and as a result, DPC was generated at 21.5 % (4.89 g) based on phenol. In this case, theoretically, the amount of water to be formed was 0.412 g. The thus-obtained reaction mixture solution was introduced into a flash tank made of stainless steel having an empty volume of 100 mL. The operation temperature and pressure during the introduction was set at 80.0°C and 0.100 MPa. Further, a liquid from a bottom of the tank was introduced into other flash tank made of stainless steel having an internal volume of 100 mL. The operation temperature and pressure during the introduction was set at 80.0°C and 0.00980 MPa. As a result of the above operation, the content of formed water in the obtained bottom liquid came to be 0.0141 g, which was 3.42 % by weight based on the amount of water that was to be theoretically formed. Further, the bottom liquid had a DPC concentration of 23.7 % by weight. To the above bottom liquid was added 15.0 g of 1,2-dimethoxyethane ($\delta_s$ = 7.48, melting point: -58.0°C), the mixture was introduced into a cylindrical vertical glass container (empty volume 100 mL), and the operation of crystallization thereof was carried out. While the bottom liquid before addition of the solvent had a temperature of 70.0°C, it was cooled to 25.0°C at the time of introduced into the crystallizer due to addition of the solvent and standing thereof at room temperature. The bottom liquid in the crystallizer was further cooled to 1.00°C. Upon completion of crystallization, a crystal and a residue were withdrawn from an outlet in the lower portion of the container, and the crystal was separated through a membrane filter. The crystal obtained had a weight of 4.05 g. DPC contained in the obtained crystal was quantitatively determined by gas chromatography, and as a result, the obtained crystal had a DPC concentration of 96.2 % by weight. The recovery of DPC was therefore 79.7 % by weight. Mother liquor obtained after the above filtering was quantitatively analyzed in various ways, such as gas chromatography, liquid chromatography, ICP, and the like. As a result, the mother liquor contained 15.4 g of phenol (recovery of 98.1 % by weight based on the total amount obtained by deducting an amount of phenol consumed in the reaction from an amount of phenol in the reaction mixture solution), 12.2 g of 1,2-dimethoxyethane, 0.0198 g of palladium acetylacetonate (recovery of 98.9 % by weight based on the charged amount), 0.0312 g of manganese acetylacetonate (recovery of 89.1 % by weight based on the charged amount), 0.0457 g of heteropolyacid (tetra-n-butyl silitungsto-11-molybdate ammonium salt) (recovery of 84.6 % by weight based on the charged amount), and 0.395 g of tetrabutylammonium bromide (recovery of 98.8 % by weight based on the charged amount). Further, a solution containing the catalyst, obtained upon completion of the crystallization, was recycled, to show that DPC was stably produced.

Example 7

Example 6 was repeated except that the amount of 1,2-dimethoxyethane ($\delta_s$ = 7.48, melting point: -58.0°C) to added after the operation of flash separation was changed to 10.0 g. A crystal obtained had a weight of 4.24 g, and DPC had a concentration of 88.2 % by weight. The recovery of DPC was therefore 76.5 % by weight. Further, a mother liquor contained 14.0 g of phenol (recovery of 89.2 % by weight based on the total amount obtained by deducting an amount of.phenol consumed in the reaction from an amount of phenol in the reaction mixture solution), 7.76 g of 1,2-dimethoxyethane, 0.0172 g of palladium acetylacetonate (recovery of 86.0 % by weight based on the charged amount), 0.0283 g of manganese acetylacetonate (recovery of 80.9 % by weight based on the charged amount), 0.0409 g of heteropolyacid (tetra-n-butyl silitungsto-11-molybdate ammonium salt) (recovery of 75.7 % by weight based on the charged amount), and 0.383 g of tetrabutylammonium bromide (recovery of 95.8 % by weight based on the charged amount). Further, a solution containing the catalyst, obtained upon completion of the crystallization, was recycled, to show that DPC was stably produced.

**Claims**

1. A method of isolating and recovering an aromatic carbonate from a solution (A), of an aromatic carbonate of the following formula (I),

$$R\text{-}O\text{-}CO\text{-}O\text{-}R \qquad\qquad (I)$$

   wherein R is an aromatic hydrocarbon group having 6 to 15 carbon atoms, the aromatic hydrocarbon group having a substituent or having no substituent,
   and an aromatic hydroxy compound of the following formula (II),

$$R\text{-}OH \qquad\qquad (II)$$

   wherein R is as defined in the formula (I),
   which comprises adding a solvent (B) having a solubility parameter ($\delta_s$) in the range of from 7.0 to 10.0 to the solution (A) selectively precipitating the aromatic carbonate in said solution (A), and then recovering the aromatic carbonate.

2. The method of isolating and recovering an aromatic carbonate as recited in claim 1, wherein said solution (A) has an aromatic carbonate concentration in the range of from 5 to 95 % by weight.

3. The method of isolating and recovering an aromatic carbonate as recited in claim 1, wherein said solution (A) has an aromatic carbonate concentration in the range of from 7 to 70 % by weight.

4. The method of isolating and recovering an aromatic carbonate as recited in claim 1, wherein said solvent (B) is at least one selected from the group consisting of aliphatic and alicyclic hydrocarbons having 5 to 8 carbon atoms, an aromatic hydrocarbon having 6 to 8 carbon atoms, aliphatic and alicyclic ethers having 3 to 6 carbon atoms, aliphatic and alicyclic ketones having 3 to 6 carbon atoms and an aliphatic alcohol having 1 to 4 carbon atoms.

5. The method of isolating and recovering an aromatic carbonate as recited in claim 1, wherein said solvent (B) is at least one selected from the group consisting of cyclohexane, toluene, xylene, diethyl ether, tetrahydrofuran, 1,2-dimethoxyethane, methyl-t-butyl ether, acetone, methyl ethyl ketone, methyl isobutyl ketone, methyl alcohol, ethyl alcohol and isopropyl alcohol.

6. The method of isolating and recovering an aromatic carbonate as recited in claim 1, wherein said solvent (B) is added to the solution (A) in such an amount ratio that the following equations (III) and (IV) are satisfied,

$$\delta_{sah} = \delta_s W_s/(W_s+W_{ah})+\delta_{ah}W_{ah}/(W_s+W_{ah}) \qquad\qquad (III)$$

$$\delta_{dac} - \delta_{sah} \geqq 0.6 \qquad (IV)$$

wherein symbols have the following meanings,

$\delta_s$: Solubility parameter of solvent (B)
$\delta_{ah}$: Solubility parameter of aromatic hydroxy compound
$\delta_{sah}$: Solubility parameter of solution (A)
$\delta_{dac}$: Solubility parameter of aromatic carbonate
$W_s$: Amount (weight) of solvent (B)
$W_{ah}$: Amount (weight) of aromatic hydroxy compound in solution (A).

7. The method of isolating and recovering an aromatic carbonate as recited in claim 1, wherein the aromatic hydroxy compound is phenol and the aromatic carbonate is diphenyl carbonate.

8. A method for producing an aromatic carbonate of the following formula (I),

$$\text{R-O-CO-O-R} \qquad (I)$$

wherein R is an aromatic hydrocarbon group having 6 to 15 carbon atoms, the aromatic hydrocarbon group having a substituent or having no substituent,
by reacting an aromatic hydroxy compound of the following formula (II),

$$\text{R-OH} \qquad (II)$$

wherein R is as defined in the formula (I),
with carbon monoxide and molecular oxygen in the presence of a catalyst,
the method further comprising adding a solvent (B) having a solubility parameter $\delta_s$ in the range of from 7.0 to 10.0 to a reaction mixture solution (A') obtained by said reaction, selectively precipitating the aromatic carbonate in said reaction mixture solution (A') and then, recovering the aromatic carbonate.

9. The method for producing an aromatic carbonate as recited in claim 8, wherein a solution (C) containing a catalyst obtained after the selective precipitation and recovery of the aromatic carbonate in said reaction mixture solution (A') is recycled to a reaction.

10. The method for producing an aromatic carbonate as recited in claim 8, wherein said catalyst is a catalyst system comprising (a) a platinum group metal or a platinum group metal compound, (b) a redox agent and (c) a quaternary ammonium salt or a quaternary phosphonium salt.

11. The method for producing an aromatic carbonate as recited in claim 10, wherein the (a) platinum group metal or the platinum group metal compound is palladium or a palladium compound.

12. The method for producing an aromatic carbonate as recited in claim 10, wherein the (b) redox agent is at least one selected from the group consisting of manganese, cobalt, copper, cerium, lead and compounds of these.

13. The method for producing an aromatic carbonate as recited in claim 10, wherein the quaternary ammonium salt is tetraalkylammonium halide.

14. The method for producing an aromatic carbonate as recited in claim 8, wherein the reaction is carried out under current of the carbon monoxide and the molecular oxygen while partial pressures of these gases are maintained at constant rates.

15. The method for producing an aromatic carbonate as recited in claim 8, wherein said reaction mixture solution (A') has an aromatic carbonate concentration in the range of from 5 to 95 % by weight.

**16.** The method for producing an aromatic carbonate as recited in claim 8, wherein said solvent (B) is at least one selected from the group consisting of aliphatic and alicyclic hydrocarbons having 5 to 8 carbon atoms, an aromatic hydrocarbon having 6 to 8 carbon atoms, aliphatic and alicyclic ethers having 3 to 6 carbon atoms, aliphatic and alicyclic ketones having 3 to 6 carbon atoms and an aliphatic alcohol having 1 to 4 carbon atoms.

**17.** The method for producing an aromatic carbonate as recited in claim 8, wherein said solvent (B) is at least one selected from the group consisting of cyclohexane, toluene, xylene, diethyl ether, tetrahydrofuran, 1,2-dimethoxyethane, methyl-t-butyl ether, acetone, methyl ethyl ketone, methyl isobutyl ketone, methyl alcohol, ethyl alcohol and isopropyl alcohol.

**18.** The method for producing an aromatic carbonate as recited in claim 8, wherein the aromatic hydroxy compound is phenol and the aromatic carbonate is diphenyl carbonate.

**19.** A method for producing an aromatic carbonate of the following formula (I),

$$R\text{-}O\text{-}CO\text{-}O\text{-}R \qquad\qquad (I)$$

wherein R is an aromatic hydrocarbon group having 6 to 15 carbon atoms, the aromatic hydrocarbon group having a substituent or having no substituent,
by reacting an aromatic hydroxy compound of the following formula (II),

$$R\text{-}OH \qquad\qquad (II)$$

wherein R is as defined in the formula (I),
with carbon monoxide and molecular oxygen in the presence of a catalyst,
the method comprising evaporating water in a reaction mixture solution (A') obtained by said reaction, under heat insulation by flashing said solution, to obtain a solution (A"), and isolating and recovering an aromatic carbonate from the solution (A").

**20.** The method for producing an aromatic carbonate as recited in claim 19, wherein a solvent (B) having a solubility parameter $\delta_s$ in the range of from 7.0 to 10.0 is added to said solution (A"), and an aromatic carbonate in said solution (A") is selectively precipitated and then separated and recovered.

**21.** The method for producing an aromatic carbonate as recited in claim 19, wherein a solvent (B) having a solubility parameter $\delta_s$ in the range of from 7.0 to 10.0 is added to said solution (A"), an aromatic carbonate in said solution (A") is selectively precipitated and then separated and recovered, and a solution (C) containing the catalyst, obtained separately, is recycled to a reaction.

**22.** The method for producing an aromatic carbonate as recited in claim 19, wherein said reaction mixture solution (A') is flashed under a temperature condition of from 0 to 100°C.

**23.** The method for producing an aromatic carbonate as recited in claim 19, wherein said reaction mixture solution (A') is flashed under a pressure condition of 1 MPa or lower.

**24.** The method for producing an aromatic carbonate as recited in claim 19, wherein said catalyst is a catalyst system comprising (a) a platinum group metal or a platinum group metal compound, (b) a redox agent and (c) a quaternary ammonium salt or a quaternary phosphonium salt.

**25.** The method for producing an aromatic carbonate as recited in claim 19, wherein said solution (A") has an aromatic carbonate concentration in the range of from 5 to 95 % by weight.

**26.** The method for producing an aromatic carbonate as recited in claim 19, wherein said solvent (B) is at least one selected from the group consisting of aliphatic and alicyclic hydrocarbons having 5 to 8 carbon atoms, an aromatic hydrocarbon having 6 to 8 carbon atoms, aliphatic and alicyclic ethers having 3 to 6 carbon atoms, aliphatic and alicyclic ketones having 3 to 6 carbon atoms and an aliphatic alcohol having 1 to 4 carbon atoms.

**27.** The method for producing an aromatic carbonate as recited in claim 19, wherein said solvent (B) is at least one selected from the group consisting of cyclohexane, toluene, xylene, diethyl ether, tetrahydrofuran, 1,2-dimethoxyethane, methyl-t-butyl ether, acetone, methyl ethyl ketone, methyl isobutyl ketone, methyl alcohol, ethyl alcohol and isopropyl alcohol.

**28.** The method for producing an aromatic carbonate as recited in claim 19, wherein the aromatic hydroxy compound is phenol and the aromatic carbonate is diphenyl carbonate.

**EP 1 270 541 A1**

| INTERNATIONAL SEARCH REPORT | International application No. |
|---|---|
| | PCT/JP01/02925 |

**A. CLASSIFICATION OF SUBJECT MATTER**
Int.Cl⁷   C07C68/08, 69/96

According to International Patent Classification (IPC) or to both national classification and IPC

**B. FIELDS SEARCHED**

Minimum documentation searched (classification system followed by classification symbols)
Int.Cl⁷   C07C68/08, 69/96

Documentation searched other than minimum documentation to the extent that such documents are included in the fields searched

Electronic data base consulted during the international search (name of data base and, where practicable, search terms used)
CAPLUS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | Chemical Abstracts, 1975, Vol.83, the abstract No.96718c,<br>CS, 157282, B (Beranek,J.),<br>15 April, 1975 (15.04.75) | 1-7<br>8-18,20,21 |
| X<br>Y | EP, 561363, A2 (IDEMITSU PETROCHEMICAL CO.LTD.),<br>22 September, 1993 (22.09.93),<br>page 6, lines 27 to 39; page 9, lines 34 to 50<br>& JP, 5-262872, A<br>Par. Nos. [0041], [0055] to [0056]<br>& US, 5349042, A      & US, 5459225, A | 1-7<br>8-18,20,21 |
| Y | EP, 583938, A1 (GENERAL ELECTRIC COMPANY),<br>23 February, 1994 (23.02.94),<br>Claims; Column 6, lines 32 to 33<br>& JP, 6-184057, A<br>Claims; Column 8, lines 14 to 15<br>& US, 5312955, A      & DE, 69315012, E<br>& ES, 2108827, T3 | 8-18 |
| X<br>Y | US, 5625091, A (Buysch et al.),<br>29 April, 1997 (29.04.97),<br>Claims; Column 1, lines 4 to 13 | 19,22-25,28<br>20,21 |

| ☒ Further documents are listed in the continuation of Box C. | ☐ See patent family annex. |
|---|---|

| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>24 May, 2001 (24.05.01) | Date of mailing of the international search report<br>05 June, 2001 (05.06.01) |
|---|---|
| Name and mailing address of the ISA/<br>Japanese Patent Office | Authorized officer |
| Facsimile No. | Telephone No. |

Form PCT/ISA/210 (second sheet) (July 1992)

15

EP 1 270 541 A1

## INTERNATIONAL SEARCH REPORT

International application No.

PCT/JP01/02925

C (Continuation).  DOCUMENTS CONSIDERED TO BE RELEVANT

| Category* | Citation of document, with indication, where appropriate, of the relevant passages | Relevant to claim No. |
|---|---|---|
| | & JP, 9-12513, A<br>Claims; Par. No. [0001]<br>& EP, 749955, A1     & DE, 19523390, A1<br>& CA, 2179581, A     & KR, 97001299, A<br>& TW, 349089, A     & CN, 1143071, A<br>& ES, 2151108, T3 | |

Form PCT/ISA/210 (continuation of second sheet) (July 1992)

## INTERNATIONAL SEARCH REPORT

| International application No. |
|---|
| PCT/JP01/02925 |

**Box I    Observations where certain claims were found unsearchable (Continuation of item 1 of first sheet)**

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.: 26,27
   because they relate to parts of the international application that do not comply with the prescribed requirements to such an extent that no meaningful international search can be carried out, specifically:

   The subject matters of claims 26 and 27 pertain to "the solvent (B) described in claim 19." However, the subject matters of claims 26 and 27 are unclear because the solvent (B) is not described in claim 19.

3. ☐ Claims Nos.:
   because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

A matter common to two groups of inventions, i.e., one consisting of claims 1 to 18, 20, and 21 and one consisting of claims 19, 22 to 25, and 28, is "a method of recovering an aromatic carbonate represented by the formula (I) from a solution (A) or (A') of an aromatic hydroxy compound represented by the formula (II) which contains the aromatic carbonate." However, this matter is not novel because it is disclosed in JP, 6-172270, A (General Electric Company), 21 June, 1994 (21.06.94). Consequently, the common matter is not a special technical feature and, hence, the two groups of inventions are not considered to be so linked as to form a single general inventive concept.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.
☒    No protest accompanied the payment of additional search fees.

Form PCT/ISA/210 (continuation of first sheet (1)) (July 1992)